# EUROPÄISCHE PATENTSCHRIFT

(11) **EP 1 797 422 B1**
(45) Veröffentlichungstag und Bekanntmachung des Hinweises auf die Patenterteilung: **13.04.2011**
(21) Anmeldenummer: 05782854.3
(22) Anmeldetag: 25.08.2005
(51) Int. Cl.: G01N 33/18, C02F 1/00, C02F 103/14

(54) **VERFAHREN UND VORRICHTUNG ZUR HERSTELLUNG WÄSSRIGER SPÜLFLÜSSIGKEITEN DURCH WIEDERAUFBEREITUNG GEBRAUCHTER SPÜLFLÜSSIGKEITEN**
METHOD AND DEVICE FOR THE PRODUCTION OF AQUEOUS RINSING LIQUIDS BY RECYCLING USED RINSING LIQUIDS
PROCEDE ET DISPOSITIF POUR PRODUIRE DES LIQUIDES DE RINÇAGE AQUEUX, PAR RECYCLAGE DE LIQUIDES DE RINÇAGE USES

(30) Priorität: 28.09.2004 DE 102004047433; 29.10.2004 DE 202004016890 U
(43) Veröffentlichungstag der Anmeldung: 20.06.2007
(73) Patentinhaber: Baumann, Didda Maria Janina, 37293 Herleshausen (DE)
(72) Erfinder: BAUMANN, Didda Maria Janina, 37293 Herleshausen (DE); BAUMANN, Walter, 32793 Herleshausen (DE)
(74) Vertreter: Strych, Werner Maximilian Josef
(86) Internationale Anmeldenummer: PCT/EP2005/009200
(87) Internationale Veröffentlichungsnummer: WO 2006/034761

(56) Entgegenhaltungen:
- GB-A- 2 062 223
- US-B1- 6 310 104
- DATABASE WPI Section Ch, Week 200256 Derwent Publications Ltd., London, GB; Class A88, AN 2002-522612 XP002359786 -& JP 2002 079263 A (HAKUTO KK) 19. März 2002 (2002-03-19)
- PATENT ABSTRACTS OF JAPAN Bd. 2003, Nr. 03, 5. Mai 2003 (2003-05-05) -& JP 2002 320975 A (SUMITOMO HEAVY IND LTD), 5. November 2002 (2002-11-05)

## Beschreibung

Die vorliegende Erfindung betrifft Verfahren und Vorrichtung zur Herstellung wässriger Spülflüssigkeiten für das Spülen von Lackieranlagen für Wasserlacke durch Wiederaufbereitung der gebrauchten Spülflüssigkeiten. Dabei werden das erzeugte Recyclat und seine während des Wiederaufbereitungsprozesses entstehenden Teilfraktionen laufend auf das Vorhandensein lackunverträglicher Kontaminationen untersucht. Das Untersuchungsergebnis wird unmittelbar zur Prozeßsteuerung verwendet.

Die erzeugten, zur Wiederverwendung vorgesehenen Recyclate können damit von Beimengungen, die die Lackverträglichkeit der gewonnenen Recyclate stören, freigehalten werden. Mit Hilfe der erfindungsgemäßen schnellen und treffsicheren Reinheitsprüfung kann die Wiederaufbereitungsanlage optimal eingestellt und während des Betriebes laufend überwacht und selbsttätig zur Prozeßoptimierung nachgeregelt werden.

Als Beispiel für die technische und wirtschaftliche Bedeutung der hier angesprochenen wässrigen Spülflüssigkeiten sei die Automobilindustrie und deren Zulieferindustrie genannt. Hier werden bereits in erheblichem Umfang und weiter zunehmend Wasserlacke eingesetzt. Wegen der wachsenden Vielfalt der auf dem Automobilmarkt angebotenen Farbvarianten müssen die Lackieranlagen der Fahrzeughersteller in immer kürzer werdenden Intervallen von einer Farbe auf eine andere Farbe umgestellt werden. Dabei werden vor jedem Farbwechsel alle vom Farbwechsel betroffenen Teile der Rohrleitungen und die Glocken (Sprühverteiler) in den Lackierstraßen mit einer wässrigen Spülflüssigkeit gespült. Aber auch ohne Farbwechsel müssen die Lackiersysteme in einem bestimmten Turnus mit der Spülflüssigkeit gespült werden, um die geforderte hohe Qualität der lackierten Karossenoberflächen dauerhaft zu gewährleisten.

Moderne Lackieranlagen der Automobilindustrie verbrauchen im Durchschnitt ca. 4 Liter Spülflüssigkeit je lackierter Karosse. Die in der Lackieranlage nach dem Spülen anfallende gebrauchte Spülflüssigkeit wird gesondert aufgefangen. Sie kann dann bis zu 40 Gew.-% Lack enthalten.

Die frische Spülflüssigkeit besteht in der Regel aus vollentsalztem Wasser (VE-Wasser), dem üblicherweise zwischen ca. 10 Gew.-% und ca. 30 Gew.-% organische Lösemittel, sowie häufig, in kleinen Mengen gewisse Tenside zugesetzt sind.

Diese Spülflüssigkeit muss beim Spülen den Lack in den Rohrleitungen und an den Glocken (Sprühverteiler) sowohl im frischen als auch im etwas angetrockneten Zustand möglichst vollständig ablösen. Da am Ende der Spülung Reste der Spülflüssigkeit in den Rohrleitungen verbleiben, sich beim Wiederbeginn des Lackierens mit dem Lack vermischen und so auf die Karossenoberfläche gelangen, darf die Spülflüssigkeit die Qualität und das Aussehen der Lackierung, an die sehr hohe Anforderungen gestellt werden, in keiner Weise negativ beeinflussen.

Es ist bekannt, dass beim Lackieren durch lokale Inhomogenitäten der Oberflächenspannung oder des Benetzungsverhaltens im noch flüssigen Lack Verlaufsstörungen ausgelöst werden, die bei der Erstarrung zum Lackfilm zu sichtbaren Oberflächenschäden führen. Eine typische Erscheinung ist die Bildung von Kratern auf der Lackoberfläche, die zur Folge haben, dass die verlangte optische Qualität der Karosse nicht erreicht wird und die Karosse kostenintensiv nachgearbeitet werden muss oder zum Ausschuss wird. Derartige sichtbare Oberflächenfehler können in der Serienfertigung großen Schaden verursachen.

Zu den Stoffen, die zu den genannten Effekten führen können, gehören beispielsweise solche aus den Stoffgruppen der Fette, Öle, Kohlenwasserstoffe, Silikone, Weichmacher und andere. Diese können auf dem Untergrund vorhanden sein oder mit ungleichmäßiger Verteilung oder falscher Dosierung mit dem Lack aufgetragen werden. Krater können auch entstehen, wenn die aufgetragene Lackschicht durch Partikel mit niedriger Oberflächenspannung aus der Luft verunreinigt wird. Störende Verunreinigungen können zum Beispiel auch durch den eigenen oder fremden Spritznebel übertragen werden.

Es ist auch bekannt, dass Wasserlacke in bezug auf die genannten Fehler deutlich empfindlicher sind als die bisher verwendeten Lösemittellacke. Kontaminierende Stoffe können bereits bei Beimengungen im ppm-Bereich zu nicht tolerierbaren Lackfehlern führen.

Jeder der hier in Betracht zu ziehenden Wasserlacke enthält zur Optimierung von Untergrundhaftung, Verlaufsverhalten, Schaumverhalten etc Lösemittel und Additive, die neben anderen Eigenschaften auch Viscosität und Oberflächenspannung während des Filmbildungsprozesses gewollt beeinflussen, die aber bei falscher Dosierung oder bei nicht ausreichend homogener Verteilung auch die vorgenannten Störungen der Oberfläche des sich bildenden Lackfilmes hervorrufen können.

Mit dem Lack gelangen die genannten Stoffe beim Spülen der Lackieranlage in die gebrauchte Spülflüssigkeit, die zur Wiederverwendung in der Lackieranlage aufbereitet werden soll. Der Prozeß der Wiederaufbereitung muss somit unter allen Umständen sicherstellen, dass diese störenden Stoffe erkannt, isoliert und abgetrennt werden.

Eine derart kontaminierte Spülflüssigkeit kann Oberflächenstörungen, wie zum Beispiel Krater, verursachen.

An einen Wiederaufbereitungsprozeß für gebrauchte Spülflüssigkeiten für Wasserlacke werden die folgenden Anforderungen gestellt:
- Die mit der ursprünglich eingesetzten frischen Spülflüssigkeit eingebrachten organischen Lösemittel sollen möglichst weitgehend zur Wiederverwendung zurückgewonnen werden.
- Das aus der lackhaltigen, gebrauchten Spülflüssigkeit stammende Wasser soll weitgehend zur Wiederverwendung zurückgewonnen werden, da die umweltgerechte Entsorgung wegen der enthaltenen restlichen Lösemittel kostenträchtig ist.
- Die Inhaltstoffe des in der gebrauchten Spülflüssigkeit enthaltenen Lackes wie Bindemittel, Pigmente, aber auch verschiedene Lösemittel und Additive müssen weitestgehend abgetrennt und ausgeschieden werden, weil diese Stoffe bei der vielmaligen Wiederaufbereitung im Recyclat systematisch angereichert würden.

Eine solche Anreicherung ist besonders schädlich, weil sich unter den Lösemitteln und Zusatzstoffen aus dem Lack auch solche Stoffe befinden, die schon in sehr geringer Beimengung die Lackverträglichkeit des Recyclates empfindlich stören und die damit das Recyclat unbrauchbar machen können.

Erschwerend kommt bei der Wiederaufbereitung der gebrauchten Spülfilüssigkeiten hinzu, dass in der Regel weder dem Betreiber der Lackieranlage noch dem Wiederaufbereiter der gebrauchten Spülflüssigkeit durch den Lackhersteller die genaue Rezeptur der Lacke offenbart wird und ihm damit die genaue Identität der Inhaltstoffe nicht bekannt ist. Das ist besonders bei den in sehr geringen Zusätzen enthaltenen Additiven kritisch, weil diese zudem kurzfristig wechseln können. Dazu kommt weiterhin, dass zum Beispiel in einem Automobilwerk Lacke von verschiedenen Herstellern in den gleichen Lackieranlagen eingesetzt werden und dass das Automobilwerk aus betrieblichen Gründen in der Regel für mehrere Wasserlackanlagen nur eine Sorte Spülflüssigkeit verwendet.

Diejenigen mit dem Lack in kleinen Mengen in die gebrauchte Spülflüssigkeit eingetragenen Lösemittel und Additive, die die Lackverträglichkeit stören, deren genaue stoffliche Zusammensetzung aber nicht bekannt ist, müssen bei der Wiederaufbereitung erkannt werden, damit sie abgetrennt und ausgeschleust werden können. Nur so kann die Qualität des Recyclates sichergestellt werden.

Die Anwesenheit kontaminiererider Lösemittel und Additive, also solcher, die die Lackverträglichkeit stören, wird in einer Spülflüssigkeit nach dem Stand der Technik durch eine Prüfung ermittelt, bei der einer ausgesuchten Lackprobe eine gewisse Menge, üblicherweise 5 Gew.-% der Spülflüssigkeit zugemischt werden. Mit diesem Gemisch wird ein Probeblech lackiert und nach dem Trocknen optisch auf die Qualität der lackierten Oberfläche geprüft.

Dieses Prüfverfahren eignet sich wegen seines Zeitbedarfes naturgemäß nicht für eine laufende betriebliche Überwachung. Außerdem ist das Prüfergebnis wegen der kleinen Prüffläche in aller Regel zu grob, um es auf die Qualitätsanforderungen einer Autokarosse zu übertragen.

Eine chemisch-analytische Prüfung der Spülflüssigkeit auf ihre Inhaltstoffe, beispielsweise durch Gaschromatographie, ist für eine betriebliche Überwachung nur dann brauchbar, wenn nach vorher bekannten Substanzen gesucht werden kann. In der Regel ist aber vorher nicht bekannt, nach welchen Substanzen im einzelnen gesucht werden soll.

Wegen der aufgeführten Probleme beim derzeitigen Stand der Technik ist die Wiederaufbereitung gebrauchter Spülflüssigkeiten aus Wasserlacklackierbetrieben und die Wiederverwendung der Recyclate im Lackierbetrieb mit erheblichen Qualitätsrisiken verbunden. Deshalb werden die gebrauchten Spülflüssigkeiten heute in den meisten Fällen entweder mit beträchtlichen Kosten umweltverträglich entsorgt oder sie werden zu Ausgleich des Wasserhaushaltes dem Wasserumlaufsystem der Lackierkabinen zugesetzt. Dieser Weg führt aber zur Emission der in der Spülflüssigkeit enthaltenen organischen Lösemittel in die Atmosphäre.

In beiden Fällen gehen die in der frischen Spülflüssigkeit enthaltenen organischen Lösemittel verloren und müssen durch Neuware ersetzt werden.

Somit kann die derzeit übliche Handhabung hinsichtlich Wirtschaftlichkeit, Umweltverträglichkeit und Resourcenschonung in den meisten Fällen nicht wirklich befriedigen.

Aus JP 2002 079263 A (Hakuto KK) ist ein Verfahren zur Herstellung wässriger Spülflüssigkeiten für das Spülen von Lackieranlagen für Wasserlacke durch Wiederaufbereitung der gebrauchten Spülflüssigkeiten und Überwachung des Recyclates auf das Vorhandensein von störenden Kontaminationen durch lackunverträgliche Begleitsubstanzen bekannt. JP-2002 079263 offenbart den Oberbegriff des Anspruchs 1, bzw. des Anspruchs 11.

Die Aufgabe dieser Erfindung ist es, ein Verfahren und eine Vorrichtung zur Verfügung zu stellen, mit deren Hilfe schnell und treffsicher festgestellt werden kann, ob ein Recyclat oder eine seiner Teilfraktionen, die während des Wederaufbertungprozesses entsteht, solche Kontaminationen, die die Lackverträglichkeit stören, enthält.

Mit Hilfe der schnellen Erkennung von störenden Kontaminationen, auch wenn nicht bekannt sein muss, um welche Stoffe es sich im einzelnen genau handelt, können bei Beginn des Wiederaufbereitungsbetriebes die zweckmäßigen Lagen der Trennschnitte festgelegt und dann im laufenden Betrieb korrigiert werden. Die Trennschnitte werden dabei so gelegt, dass die Kontaminationen gezielt in bestimmte Fraktionen sortiert werden, die ihrerseits möglichst wenige wertvolle und wieder verwertbare Stoffe enthalten. Diese Frakionen werden dann nach wirtschaftlichen Gesichtspunkten weiterbehandelt oder ausgeleitet.

Durch eine ständige Überwachung der bei der Wiederaufbereitung der Spülflüssigkeit entstehenden Teilfraktionen kann das erfindungsgemäße Erkennungsverfahren selbsttätig korrigierend in den Prozeß eingreifen. Zunächst wird automatisch der Zulauf der kontaminierten Menge zum Reintank gesperrt. Weiterhin kann eine Signalgabe erfolgen und eine Korrektur von Einstellungen des Prozesses ausgelöst werden. Eine Ablieferung von nicht qualitätsgerechter Ware wird so ausgeschlossen.

Der Prozeß zur Wiederaufbereitung gebrauchter Spülflüssigkeiten selbst ist ein Trennprozeß nach dem Stand der Technik durch Destillation, Membranverfahren und andere Verfahren in geeigneter Ausgestaltung und Kombination. Art und Menge der störenden Kontaminationen in der eingesetzten gebrauchten Spülflüssigkeit sind zunächst nicht bekannt und können außerdem kurzfristig wechseln. Mit Hilfe des nachstehend beschriebenen erfindungsgemäßen Verfahrens und der erfindungsgemäßen Vorrichtung kann während der Wiederaufbereitung festgestellt werden, ob das Recyclat oder eine aus dem Prozeß entnommene Teilfraktion störende Kontaminationen enthält oder nicht.

Mit dieser Prüfung können die Betriebsparameter, beispielsweise Temperaturen, zur Einstellung der Trennschnitte bei der Wiederaufbereitung so eingerichtet werden, dass die Teilfraktionen während der Wiederaufbereitung jeweils frei von Kontaminationen sind und so im Recyclat verwendet werden können. Die kontaminierenden Substanzen können gezielt in gewissen Teilfraktionen konzentriert und mit diesen ausgeschleust werden.

Ein unerwartetes kurzfristiges Auftreten von Kontaminationen in einer Fraktion infolge von Veränderungen im Aufgabegut kann durch die automatische laufende Prüfung mit Hilfe von Vorrichtung und Verfahren dieser Erfindung sofort erkannt und das verunreinigte Produkt selbsttätig abgeleitet werden. Damit wird die Qualität des erzeugten Recyclates im durchgehenden Betrieb gesichert.

Diese Aufgaben erfüllt die vorliegende Erfindung.

Überraschenderweise wurde gefunden, dass solche Substanzen, die in der Spülflüssigkeit die Lackverträglichkeit stören, die Eigenschaft haben, auch bei sehr geringer Konzentration in der geprüften Flüssigkeit eine deutliche Trübung zu erzeugen, wenn der Wassergehalt der Mischung einen bestimmten Wert erreicht oder übersteigt. Dies gilt auch für Recyclatteilfraktionen, die beim Wiederaufbereitungsprozeß erzeugt werden und aus denen dann später das Recyclat zusammensesetzt wird.

Es wurde gefunden, dass klare, farblose Recyclate oder klare, farblose Teilfraktionen aus dem Wiederaufbereitungsprozeß immer dann, wenn sie bei Zumischen von VE-Wasser bis auf einen Endwassergehalt von 95 Gew.-% keine Trübung zeigten, stets frei waren von lackunverträglichen Kontaminationen. Die beschriebene, heute übliche Lackverträglichkeitsprüfung durch Lackieren von Prüfblechen zeigte stets keinerlei Oberflächenschäden der Lackschicht. Es wurde auch gefunden, dass Recyclate oder Fraktionen, die bis zu einem Wassergehalt von 95 Gew.-% keine Trübung zeigten, stets auch bei einem darüberhinausgehenden Wassergehalt klar blieben und bei der Lackverträglichkeitsprüfung keine Oberflächenschäden aufwiesen. Das zeigt, dass sie wirklich frei von Kontaminationen sind.

Somit reicht es für eine erschöpfende Prüfung gemäß der vorliegenden Erfindung aus, einer Recyclatprobe oder einer Teilfraktion, die im Originalzustand klar durchsichtig ist, zur Prüfung auf lackunverträgliche Kontaminationen gerade soviel VE-Wasser zuzusetzen, dass in der Prüfmischung ein Wassergehalt von 95 Gew.-% erreicht wird. Ein darüber hinausgehender Wasserzusatz würde keine zusätzlichen Erkenntnisse erbringen.

Proben von Recyclat oder Teilfraktionen, die im Ausgangszustand klar durchsichtig waren und die während eines portionsweisen Zusatzes von VE-Wasser mit dem zunehmenden Wassergehalt der Probeflüssigkeit zwischen dem Ausgangswert und 95 Gew.-% trüb wurden, zeigten bei der Lackverträglichkeitsprüfung durch Lackieren von Prüfblechen unterschiedlich stark sichtbare Störungen der lackierten Oberfläche (Kraterbildung).

Es wurde auch gefunden, dass Recyclate oder Teilfraktionen, die bei der Wiederaufbereitung in trübem Zustand anfallen, auch bei einem weiteren Zusatz von Wasser trüb bleiben und dass diese Produktproben bei der Lackverträglichkeitsprüfung unterschiedlich deutliche Lackunverträglichkeiten zeigen.

Vergleichsversuche zeigten, dass für die beschriebene Prüfung auf das Vorhandensein lackunverträglicher Kontaminationen anstelle von VE-Wasser auch Leitungswasser einer örtlichen Trinkwasserqualität eingesetzt werden konnte, ohne dass die Ergebnisse merkbar abwichen. Da eine solche Austauschbarkeit bei unterschiedlichen Wasserqualitäten im Einzelfall quantifiziert werden muss, beziehen sich die hier vorgelegten Zahlenwerte stets auf VE-Wasser. Die hier dargelegte Erfindung funktioniert nach den vorliegenden Ergebnissen sowohl mit VE-Wasser als auch mit Wasser einer üblichen Trinkwasserqualität. Die jeweilige genaue Prüfdosierung muss gegebenenfalls mit Hilfe von Vergleichsversuchen angepasst werden.

Es wurde weiterhin gefunden, dass Recyclate oder deren Teilfraktionen, die mit einem Ausgangswassergehalt von ≥ 85 Gew.-% klar durchsichtig vorliegen und bei Zusatz von Wasser bis zu einem erreichten Endgehalt von 93 Gew.-% Wasser keine Trübung zeigten, auch bei einem weiteren Zusatz von Wasser klar bleiben und frei sind von lackunverträglichen Kontaminationen. Das bedeutet für die betriebliche Prüfung ein Massenverhältnis von Spülflüssigkeit und zur Prüfung zugesetztem VE-Wasser von 1:1.

Recyclate oder deren Teilfraktionen, die bei einem Ausgangswassergehalt zwischen 60 Gew.-% und 85 Gew.-% klar durchsichtig vorliegen und bei Zusatz von Wasser bis zu einem Wasserendgehalt von 87 Gew.-% keine Trübung zeigen, waren frei von störenden Kontaminationen. Das bedeutet im Mittel eine Mischung von Spülflüssigkeit und zur Prüfung zugesetztem VE-Wasser im Massenverhältnis 1 : 2.

Recyclate oder deren Teilfraktionen, die bei einem Ausgangswassergehalt zwischen 60 Gew.-% und 40 Gew.-% klar durchsichtig vorliegen und bei Zusatz von Wasser bis zu einem Wasserendgehalt von 85 Gew.-% keine Trübung zeigen, waren frei von störenden Kontaminationen. Das bedeutet im Mittel eine Mischung von Spülflüssigkeit und zur Prüfung zugesetztem VE-Wasser im Massenverhältnis 1 : 3.

Recyclate oder deren Teilfraktionen, die bei einem Ausgangswassergehalt < 40 Gew.-% klar durchsichtig vorliegen und bei Zusatz von Wasser bis zu einem Wasserendgehalt von 80 Gew.-% keine Trübung zeigen, waren frei von störenden Kontaminationen. Das bedeutet im Mittel eine Mischung von Spülflüssigkeit und zur Prüfung zugesetztem VE-Wasser im Massenverhältnis 1 : 4.

Mit diesen Anhaltswerten kann für die laufende betriebliche Überwachung die der Recyclatprobe zuzusetzende Wassermenge bestimmt werden. Es ist lediglich notwendig, durch eine einfache Wasserbestimmung einen groben Anhaltswert für den Wassergehalt der zu prüfenden Spülflüssigkeit zu ermitteln. Diese Darlegung zeigt deutlich den Vorteil der vorliegenden Erfindung zur Führung des Wiederaufbereitungsprozesses. Eine genaue und zeitraubende Analytik ist nicht erforderlich.

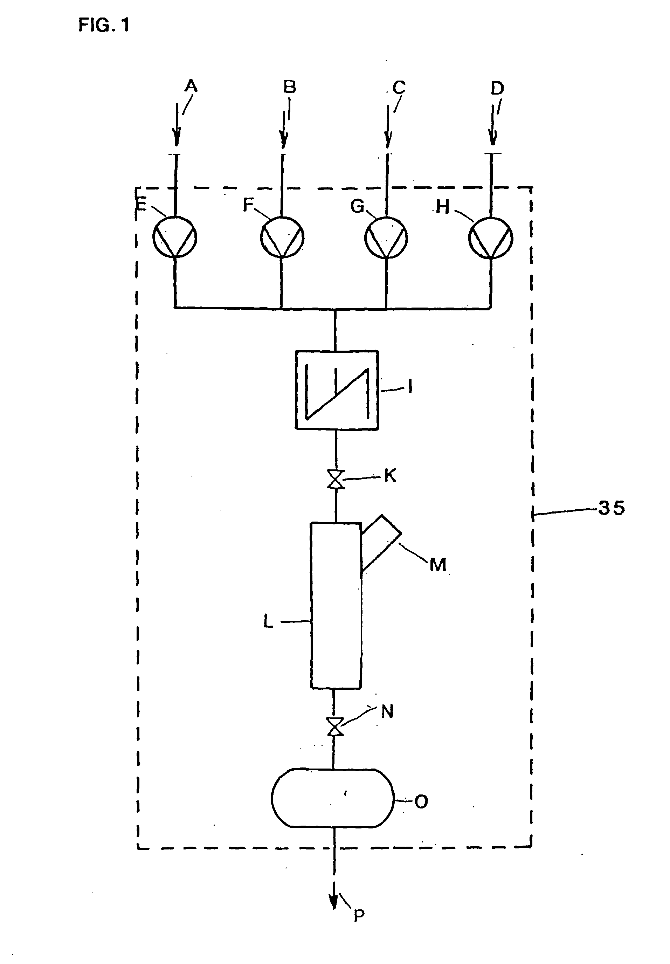

Die Vorrichtung zur erfindungsgemäßen Erkennung lackunverträglicher Kontaminationen in einer Spülflüssigkeit zeigt FIG.1 für eine beispielhaft gewählte Ausführung mit drei Dosierpumpen für drei Recyclatfraktionen und mit einer Dosierpumpe für das VE-Wasser, das zur Prüfung zugesetzt wird.

In der erfindungsgemäßen Vorrichtung werden Spülflüssigkeit und VE-Wasser durch Dosierpumpen mit jeweils voreingestellten Fördermengen vorgelegt, gemischt und mit einem handelsüblichen Trübungssensor auf Trübung untersucht. Das Meßsignal des Trübungssensors wird angezeigt und kann unmittelbar zur Prozeßsteuerung eingesetzt werden. So werden lackunverträgliche Kontaminationen schnell und sicher erkannt, ohne, dass es erforderlich ist festzustellen, um welche Stoffe es sich im einzelnen genau handelt.

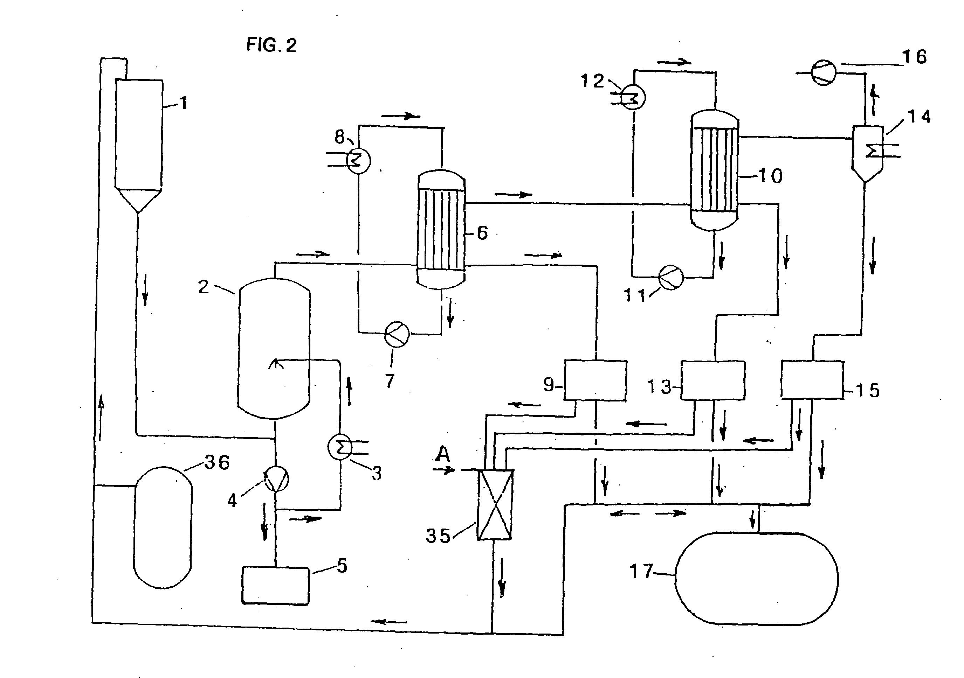

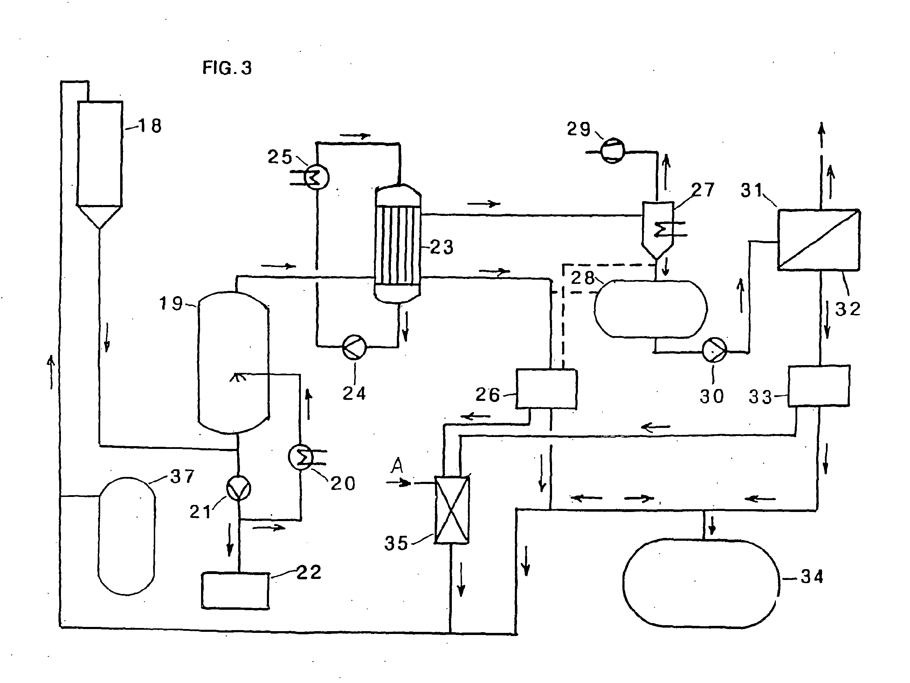

Die zu prüfende Substanze (B), (C), (D), das ist beispielsweise jeweils eine einzelne Recyclatfraktion aus dem Wiederaufbereitungsprozeß oder auch fertiges Recyclat, wird jeweils mit der voreingestellten Menge durch die Dosierpumpe (F), (G), (H) programmgesteuert in die Mischkammer (I) gefördert. Dann fördert die Dosierpumpe (E) automatisch die eingestellte Menge VE-Wasser (A) in die Mischkammer (I). Nach kurzem Mischen durch den Rührer öffnet das Ventil (K) und die Probesubstanz strömt in die Meßstrecke (L) des Trübungsmeßgerätes. Nach Messung der Trübung durch den Sensor (M) öffnet das Ventil (N) und die Probesubstanz fließt zum Auffangbehälter (O). Hier wird die gebrauchte Probesubstanz gesammelt und in Intervallen zurück zum Vorlagebehälter (1) beim Prozeß gemäß FIG.2, beziehungsweise zum Vorlagebehälter (18) beim Prozeß gemäß FIG.3 geleitet.

Bezogen auf die später noch beispielhaft beschriebene Verfahrensführung gemäß FIG.2 entspricht Probesubstanz (B) der ersten Destillatfraktion in Behälter (9), die Probesubstanz (C) entspricht der zweiten Destillatfraktion in Behälter (13) und die Probesubstanz (D) entspricht der Endfraktion in Behälter (15). (A) bezeichnet das zur Untersuchung zugesetzte VE-Wasser. Die komplette erfindungsgemäße Vorrichtung (35) ist in FIG.2 und in FIG.3 angedeutet ohne Einzelheiten dargestellt.

Bezogen auf die später noch beispielhaft beschriebene Verfahrensführung gemäß FIG.3 entspricht die Probesubstanz (B) der ersten Destillatfraktion in Behälter (26), die Probesubstanz (C) entspricht dem Permeat in Behälter (33).

Die frische Spülflüssigkeit besteht üblicherweise aus einem Gemisch verschiedener wassermischbarer organischer Lösemittel und VE-Wasser. Der Lösemittelanteil liegt üblicherweise im Bereich zwischen 10 Gew.-% und 30 Gew.-%. Als Lösemittel werden heute beispielsweise n-Propanol, Butoxyethanol, Ethanol, Butanol, Dimethylethanolamin und andere verwendet. Die Mischungsverhältnisse der Lösemittel untereinander sind sehr unterschiedlich. So findet man beispielsweise, bezogen auf den Lösemittelanteil der Spülflüssigkeit, Anteile an Butoxyethanol zwischen 100 Gew.-% und 25 Gew.-%, an n-Propanol zwischen 0 Gew.-% und 65 Gew.-% und an n-Butanol zwischen 0 gew.-% und 16 Gew.-%.

Nach dem Gebrauch enthält die aufgefangene Spülflüssigkeit zusätzlich zu den vorgenannten Komponenten der frischen Spülflüssigkeit zwischen circa 15 Gew.-% und 40 Gew.-% Lack, der aus den Rohrleitungen der Lackieranlage herausgespült wurde. Der Lack enthält seinerseits üblicherweise ca. 20 Gew.-% bis 50 Gew.-% Festkörper (Pigmente, Füllstoffe, Bindemittel), sowie Wasser und ein breites Spektrum organischer Lösemittel und Additive, wie beispielsweise Butoxyethanol, Hexoxyethanol, N-Methylpyrrolidon, Butanol, verschiedene Kohlenwasserstoffe, Silikone, Tenside und andere.

Bei der Wiederaufbereitung der gebrauchten Spülflüssigkeit sollen einerseits die aus der frischen Spülflüssigkeit stammenden Lösemittel wie n-Propanol, Butoxyethanol, Dimethylethanolamin sowie das aus dem Lack stammende Butoxyethanol möglichst weitgehend zurückgewonnen werden. Andererseits müssen die übrigen aus dem Lack stammenden, meist schwererflüchtigen Lösemittel und die Additive abgetrennt und entfernt werden, weil sie beim wiederholten Recycling angereichert würden und weil sich in dieser Stoffgruppe auch die Substanzen befinden, die, wenn sie durch die Wiederaufbereitung der gebrauchten Spülflüssigkeit zum Bestandteil der neuen, zurückgewonnenen Spülflüssigkeit werden, die Lackverträglichkeit stören und damit das Recyclat unbrauchbar machen.

Für die zur Wiederaufbereitung gebrauchter Spülflüssigkeiten erforderliche Stofftrennung eignen sich nach dem Stand der Technik insbesondere Destillations- und Membranverfahren beziehungsweise Kombinationen von beiden.

Es werden bewusst keine Verfahren in Betracht gezogen, bei denen dem Einsatzgut Hilfsstoffe zugesetzt werden, weil hier stets die Gefahr besteht, dass Reste der Hilfsstoffe im Recyclat verbleiben und dessen Lackverträglichkeit negativ beeinflussen können. Deshalb werden hier beispielsweise Verfahren der Flüssig-flüssig-Extraktion oder der Hilfsmitteldestillation nicht in Betracht gezogen.

Generell ist bei der Anwendung von Destillationsverfahren zur Wiederaufbereitung wässriger Spülflüssigkeiten zu beachten, dass diese Spülflüssigkeiten immer einen hohen Anteil an Wasser enthalten und dass die meisten der enthaltenen Lösemittel und Additive mit Wasser azeotrope Gemische bilden. Deren Siedepunkte liegen eng benachbart und bewegen sich im wesentlichen in einem engen Bereich unterhalb der Siedetemperatur des Wassers. Deshalb ist ein Destillationsverfahren mit hoher Trennschärfe erforderlich, gerade auch für solche Stoffe, die nur mit einem geringen Anteil im Promillebereich oder darunter enthalten sind, wie das bei den störenden Kontaminationen in der Regel der Fall ist.

Bei der Wiederaufbereitung wird die gebrauchte Spülflüssigkeit stets zunächst in Teilfraktionen zerlegt, die so geschnitten werden, dass in ihnen getrennt jeweils einerseits die Stoffe, die zurückgewonnen werden sollen und andererseits diejenigen, die abgetrennt und entfernt werden sollen, möglichst hoch angereichert enthalten sind. Die erstgenannten Teilfraktionen werden im Recyclat verwendet, die letztgenannten werden, je nach ihrem Anreicherungsgrad in einer weiteren Trennstufe zusätzlich aufbereitet oder sie werden, wenn wegen des zu geringen Gehaltes an wertvollen Inhaltstoffen eine weitere Aufbereitung nicht sinnvoll ist, verworfen.

Ein besonderes Problem bei der destillativen Wiederaufbereitung gebrauchter wässriger Spülflüssigkeiten sind die zahlreichen, ständig wechselnden und im einzelnen vorher nicht bekannten Bestandteile des enthaltenen Lackes. Der Lack enthält sowohl für die Wiedergewinnung wertvolle als auch störende Inhaltstoffe, die sich durch ihre azeotropen Eigenschaften gegenseitig in den resultierenden Siedelagen beinflussen. Deshalb kann nur sehr unvollkommen vorhergesagt werden, in welchen Teilfraktionen die wertvollen und in welchen die störenden Substanzen anfallen und konzentriert werden können. Hier gibt die Erfindung die Möglichkeit, einfach und schnell die mit gewissen Probeeinstellungen hergestellten Siedeschnitte jeweils empirisch auf lackunverträgliche Kontaminationen zu prüfen und die Trennschnitte der Fraktionen dann systematisch so zu verschieben, dass nichtkontaminierte Teilfraktionen zur Wiederverwendung und kontaminierte Teilfraktionen zur Weiterbearbeitung oder zur Entsorgung erzeugt werden. Dabei müssen die zur Wiederverwendung vorgesehenen Teilfraktionen frei von lackunverträglichen Kontaminationen sein. Die zum Ausschleusen vorgesehenen kontaminierten Fraktionen sollen möglichst wenig wertvolle wiederverwendbare Substanzen enthalten.

Im Folgenden werden werden beispielhaft zwei Möglichkeiten gezeigt, in welcher Weise die dargelegte Erfindung vorteilhaft industriell genutzt werden kann.

Als ein besonders vorteilhaftes Wiederaufbereitungsverfahren im Hinblick auf die vorgenannten Erfordernisse ergibt sich die Kombination der vorliegenden Erfindung mit dem Verfahren der kontinuierlichen Vakuumdestillation mit fraktionierter Kondensation gemäß EP 0812233 B1 und USPat 6,117,275 in einer Ausführung mit zwei oder mehreren geregelten Kondensationsstufen und einer ungeregelten Kondensationsendstufe, wie es nachfolgend in FIG.2, beispielhaft für die Ausgestaltung mit zwei geregelten und eine ungeregelten Kondensationsstufen, schematisch dargestellt ist.

Die gebrauchte Spülflüssigkeit fließt vom Vorlagetank (1) zum kontinuierlichen Verdampfer (2). Die Beheizung des Verdampfers kann, wie hier beispielhaft dargestellt ist, über einen Sumpfkreislauf mit regelbarer Pumpe (4) und den beheizten Wärmetauscher (3) erfolgen. Der Füllstand im Verdampfer (2) wird durch einen Füllstandsgrenzschalter konstant gehalten. Es wird jeweils gerade soviel gebrauchte Spülflüssigkeit in den Verdampfer nachgespeist, wie im betrachteten Zeitintervall verdampft ist. In periodischen Abständen wird das umlaufende Produkt des Sumpfkreislaufes, wenn es auf ca. 30 Gew.-% Festkörpergehalt (Destillationsrückstand) angereichert ist, automatisch mit Hilfe der Umlaufpumpe (4) in den Rückstandbehälter (5) gefördet. Dabei muss beim beschriebenen Verdampfersystem darauf geachtet werden, dass der aufkonzentrierte Rückstand stets fließfähig gehalten wird.

Anstelle des dargestellten Verdampfersystems ist auch der Einsatz anderer Systeme wie Dünnschichtverdampfer, Fallstromverdampfer oder Plattenverdampfer möglich.

Das gezeigte Destillationssystem kann wahlweise bei Atmosphärendruck oder bei Vakuum betrieben werden, wenn beispielsweise temperaturempfindliche Komponenten im Stoffsystem enthalten sind.

Die nichtverdampfbaren Bestandteile der gebrauchten Spülflüssigkeit, wie Pigmente, Füllstoffe und Bindemittel, die unter dem Begriff "Festkörper" zusammengefasst werden, sowie hochsiedende Stoffe, die mit Wasser oder unter sich keine niedrigsiedenden Azeotrope bilden, werden im Verdampferkreislauf angereichert, periodisch automatisch ausgeschleust und entsorgt beziehungsweise stofflich oder thermisch verwertet.

Alle den Verdampfer (2) in Dampfform verlassenden Stoffe werden zum ersten Kondensator (6) geleitet. Diese flüchtigen Bestandteile enthalten neben dem Wasser die in der gebrauchten Spülflüssigkeit enthaltenen Lösemittel und die bei den jeweiligen Verdampfungsbedingungen flüchtigen Additive. Der Kondensator (6) ist mit einem geschlossenen und geregelten Kühlkreislauf, bestehend aus der regelbaren Umlaufpumpe (7) und dem Kühler (8), ausgestattet. Die Siedelage des entstehenden Kondensates wird durch die geregelte Temperatur des Kühlmittels am Eintritt in den Kondensator (6) und durch die Fördermenge der Umlaufpumpe (7), die die Temperatur des Kühlmittels an dessen Austritt aus dem Kondensator (6) bestimmt, eingestellt. Die Austrittstemperatur der dampfförmigen Leichtsieder am Kopf des Kondensators (6) und die Austrittstemperatur des flüssigen Kondensates am Fuß des Kondensators werden auf diese Weise unabhängig voneinander eingestellt. Dieses System gestattet die Kondensation präziser Siedeschnitte.

Im ersten Kondensator (6) wird die Fraktion mit den höchsten Siedegrenzen kondensiert. Diese erste Fraktion wird im Zwischenbehälter (9) aufgefangen und in festen Zeitintervallen automatisch mit Hilfe der erfindungsgemäßen Vorrichtung (35) auf das Vorhandensein von störenden Kontaminationen untersucht. Erst nach dieser Untersuchung wird der Inhalt des Behälters (9), wenn er frei von Kontaminationen ist, zum Reintank (17) geleitet. Ist er kontaminiert, schaltet die Vorrichtung (35) selbsttätig den Weg zurück zum Vorlagebehälter (1) frei. Von dort durchläuft das Produkt nochmals die Wiederaufbereitung. Dabei wird manuell oder durch direkten Eingriff des Trübungssensors in die Prozeßsteuerung die Lage des Trennschnittes der fraktionierten Kondensation solange verändert, bis ein nichtkontaminiertes Produkt erhalten wird. Die kontaminierenden Substanzen werden, möglichst hoch angereichert, in eine dafür vorgesehene Fraktion und zum Behälter (36) geleitet. Von dort wird sie, je nach Inhaltstoffen, wahlweise weiter aufbereitet, anderweitig stofflich oder thermisch verwertet, oder entsorgt.

So ist sichergestellt, dass während des laufenden Betriebes schädliche Veränderungen der Destillatzusammensetzung, verursacht durch eine wechselnde Zusammensetzung des Aufgabegutes, sicher bemerkt werden. Das kontaminierte Produkt wird automatisch zum Redestillieren zurückgeleitet, ohne den Inhalt des Reintanks (17) zu kontaminieren.

Die Komponenten mit tieferer Kondensationstemperatur verlassen den Kondensator (6) dampfförmig und werden zum zweiten Kondensator (10) geleitet. Sein System ist mit einem geschlossenen und geregelten Kühlkreislauf, mit einer regelbaren Umlaufpumpe (11) und einem Kühler (12) ebenso aufgebaut, wie das des Kondensators (6). Im Kondensator (10) wird die zweite Fraktion kondensiert. Ihre Kondensationstemperatur liegt unter derjenigen der ersten Fraktion aus dem Kondensator (6). Die Siedelage der zweiten Fraktion wird mit der geregelten Temperatur des Kühlmittels am Eintritt in den Kondensator (10) und mit der Fördermenge der Umlaufpumpe (11) eingestellt. Die zweite Fraktion, also das Kondensat des Kondensators (10), wird in den Zwischenbehälter (13) geleitet.

Wie die erste Fraktion, wird auch die zweite Fraktion durch das erfindungsgemäße Verfahren und die Vorrichtung (35) automatisch beprobt und auf Kontamination geprüft. Ist der Inhalt des Behälters (13) frei von Kontaminationen, wird das Destillat zum Reintank (17) geleitet. Ist der Inhalt kontaminiert, läuft das Produkt zurück zum Vorlagebehälter (1) und wird nochmals, mit veränderten Einstellungen, aufbereitet. Nach der Optimierung der Einstellungen gibt es dann entweder eine Destillatfraktion zur Wiederverwendung, die zum Reintank (17) geleitet wird, oder es gibt eine Destillatfraktion, in der die Kontaminationen angereichert sind und die zum Behälter (36) geleitet wird, von wo sie weiterbearbeitet oder ausgeleitet wird.

Die im Kondensator (10) nicht kondensierten Leichtsieder verlassen den Kondensator und strömen zum Endkondensator (14), in dem bei tiefer Temperatur alle noch vorhandenen kondensierbaren Dämpfe kondensiert und in den Zwischenbehälter (15) geleitet werden. Dort wird das Kondensat durch die erfindungsgemäße Vorrichtung (35) in gleicher Weise automatisch beprobt und untersucht, wie das für die Fraktionen der Behälter (9) und (13) beschrieben ist. Ist die dritte Fraktion in Behälter (15) frei von Kontaminationen, gibt das Trübungsmeßgerät den Weg zum Reintank (17) frei. Ist die Fraktion kontaminiert, läuft das Produkt zurück zum Vorlagetank (1). Im Rahmen der Gesamtoptimierung wird, wie bereits schon beschrieben, verfahren.

Im Reintank (17) werden über einen festgelegten Zeitraum diejenigen Fraktionen gesammelt, die mit Sicherheit frei von Kontaminationen sind. Das gesammelte Produkt wird dann, beispielsweise gaschromatographisch, auf seine genaue Zusammensetzung hinsichtlich der für die frische Spülflüssigkeit spezifizierten Komponenten analysiert. Durch Hinzufügen fehlender Substanzanteile oder Verdünnen mit VE-Wasser wird die gewünschte Endzusammensetzung eingestellt und das fertige Produkt zur Verwendung in der Lackieranlage freigegeben.

Das dargelegte Wiederaufbereitungsverfahren für gebrauchte wässrige Spülflüssigkeiten, das aus der Kombination von Verfahren und Vorrichtung der vorliegenden Erfindung zur Erkennung lackunverträglicher Kontaminationen und einem für die jeweils beteiligten Stoffe geeigneten Trennverfahren besteht, kann hinsichtlich seiner Ausgestaltung den Eigenschaften der beteiligten Stoffe angepasst werden, ohne dass das Zusammenwirken der erfindungsgemäßen Erkennung der Kontaminationen und des Trennverfahrens selbst geändert wird. Dies ist nachfolgend am Beispiel der besonders vorteilhaften Kombination mit der kontinuierlichen Destillation mit fraktionierter Kondensation gemäß EP 0812233 B1 und US Pat 6,117,275 beschrieben.

Der in FIG.2 dargestellte Prozeß besteht in dieser beispielhaften Ausführung aus zwei geregelten Kondensationsstufen und einer ungeregelten Kondensationsendstufe. Je nach der Zahl und der Art der in der gebrauchten Spülflüssigkeit vorkommenden Substanzen kann es zur Optimierung des Trennergebnisses zweckmäßig sein, drei oder mehrere geregelte Kondensationsstufen und eine ungeregelte Kondensationsendstufe einzurichten. Die Beprobung und Untersuchung der Destillate der dritten oder weiteren Kondensationsstufe erfolgt in gleicher Weise wie an den Kondensationsstufen 1 (6) und 2 (10) dargestellt.

Enthält die wiederaufzubereitende gebrauchte Spülflüssigkeit gewisse Hauptlösemittel, deren Stoffeigenschaften dazu führen, dass eine befriedigende destillative Trennung von bestimmten störenden lackunverträglichen Kontaminationen nicht möglich ist, so können beispielsweise eine oder mehrere Kondensationsstufen durch geeignete Membranfilterstufen ersetzt werden. Eine solche beispielhafte Anordnung zeigt FIG.3. Das Trennverfahren besteht hier aus einer kontinuierlichen Destillation mit fraktionierter Kondensation gemäß EP 0812233 B1 und US Pat 6,117,275 in einer Ausführung mit Verdampfer, einer geregelten Kondensationsstufe und einer ungeregelten Kondensationsendstufe, wobei der Kondesationsendstufe eine Membrantrennanlage nachgeschaltet ist, die im hier dargestellten Beispiel das zurückzugewinnende Lösemittel nach dem Molgewicht von störenden Begleitstoffen trennt.

Der Wiederaufbereitungsprozeß nach FIG.3 entspricht im ersten Teil dem Prozeß nach FIG.2. Der Verdampfer (19) in FIG.3 entspricht Verdampfer (2) in FIG.2, die erste geregelte Kondensationsstufe mit Kondensator (23) in FIG.3 entspricht der ersten geregelten Kondensationsstufe mit Kondensator (6) in FIG.2, der Endkondensator (27) in FIG.3 entspricht dem Endkondensator (14) in FIG.2. An die Stelle der zweiten geregelten Kondensationsstufe mit Kondensator (10) in FIG.2 tritt in FIG.3 eine Trennstufe mit Membranverfahren (31/32), die nach dem Endkondensator angeordnet ist.

Wie FIG.3 zeigt, strömt die gebrauchte Spülflüssigkeit aus dem Vorlagebehälter (18) zum Verdampfer (19), der über den Sumpfkreislauf, bestehend aus der regelbaren Umlaufpumpe (21) und dem Durchlauferhitzer (20), beheizt wird. Der nicht verdampfbare Anteil (Festkörper) der gebrauchten Spülflüssigkeit wird so weit angereichert, dass der Rückstand fließfähig bleibt. Er wird, wie im Verfahren zu FIG.2 beschrieben, automatisch intervallweise über den Entnahmebehälter mit Schleuse (22) entnommen. Auch für den Prozeß gemäß FIG.3 kann, wie am Prozeß gemäß FIG.2 beschrieben, ein anderes Verdampfersystem, beispielsweise ein Dünnschichtverdampfer, Fallstromverdampfer oder Plattenverdampfer eingesetzt werden.

Das Dämpfegemisch aus dem Verdampfer (19) wird zum Kondensator (23) geleitet, der mit Hilfe eines geschlossenen Kühlkreislaufes gekühlt wird. Durch die Einstellung der Solltemperatur des umlaufenden Kühlmittels am Kühler (25) und die Einstellung der umlaufenden Kühlmittelmenge an der regelbaren Pumpe (24) werden die Temperaturen am Kopf des Kondensators (23) (Leichtsiederaustritt) und am Fuß des Destillators (23) (Destillataustritt) getrennt geregelt, sodaß im Sammelbehälter (26) eine präzise geschnittene Fraktion erhalten wird. Die im Kondensator (23) nicht kondensierte Leichtsiederfraktion verlässt den Kondensator (23) an dessen Kopf, wird dann im Endkondensator (27) komplett kondensiert und im Sammelbehälter (28) aufgefangen. Die Beprobung und Untersuchung der jeweiligen Destillatfraktion in den Behältern (26) beziehungsweise (28) auf lackunverträgliche Kontaminationen erfolgt durch Verfahren und Vorrichtung (35) gemäß der vorliegenden Erfindung in der gleichen Weise, wie das für den Prozess der FIG.2 beschrieben ist.

Wenn, wie das für die untersuchten Spülflüssigkeiten bisher stets gefunden wurde, die störenden Kontaminationen durch Bildung niedrigsiedender höherer Azeotrope mit Wasser und vorhandenen Lösemitteln bei der fraktionierten Kondensation besonders in der letzten, also der am niedrigsten siedenden Fraktion angereichert werden, kann durch geeignete Wahl der Grenztemperaturen die erste, also die am höchsten siedende Fraktion, kontaminationsfrei gewonnen werden. In diesem Fall wird die Einstellung der Kondensationstemperatur am Kondensator (23) der FIG.3 solange systematisch verändert, bis das Destillat im Behälter (26) nach Beprobung und Untersuchung durch die Meßeinrichtung gemäß der vorliegenden Erfindung (35) als kontaminationsfrei befunden ist. Es wird zum Reintank (34) geleitet und kann im Recyclat verwendet werden. Die niedriger siedende zweite Fraktion aus dem Endkondensator (27), die die Kontaminationen enthält, wird im Sammelbehälter (28), der der Vorlagebehälter der Membrantrennanlage (31/32) ist, aufgefangen. Von hier läuft das Produkt über die Druckpumpe (30) zur Membrantrennanlage (31), in der nach dem Stand der Technik mit Hilfe geeigneter Membranen Wasser und leichte Lösemittel als Permeat (32), sowie Kontaminationen und schwerere Lösemittel mit höherem Mol.-gewicht als Retentat (31) auseinandergetrennt werden. Bei der Auswahl einer geeigneten Membran ist die optimale Trenngrenze dann gefunden, wenn das Permeat (32) kontaminationsfrei ist, und gleichzeitig mit dem Retentat (31) wertvolle Inhaltstoffe nur in wirtschaftlich vertretbarem Umfang verloren gehen. Geprüft wird mit Verfahren und Vorrichtung der vorliegenden Erfindung (35).

Sollten in einer gebrauchten Spülflüssigkeit Kontaminationen enthalten sein, deren Kondensationstemperatur im Gemisch gleich oder höher als die des reinen Wassers ist, so werden diese Kontaminationen in der ersten Fraktion des Kondensators (23) angereichert und gelangen in den Sammeltank (26). Die Kondensationstemperatur des Kondensators (23) wird in diesem Fall so eingestellt, dass die zweite Fraktion aus Endkondensator (27) im Sammelbehälter (28) kontaminationsfrei anfällt. Die erste Fraktion aus Kondensator (23) wird, wie das in FIG.3 durch gestrichelte Linie dargestellt ist, zum Behälter (28) geleitet und von hier der Membrantrennanlage (31/32) zugeführt. Die Verbindung des Kondensators (23) zum Sammeltank (26) wird unterbrochen. Das Kondensat des Endkondensators (27), das kontaminationsfrei ist, wird zum Sammelbehälter (26) und von dort zum Reintank (34) geleitet. Der Kondensatablaß des Endkondensators (27) wird mit Behälter (26) verbunden. Diese Verbindung ist in FIG.3 durch gestrichelte Linie dargestellt. Die Verbindung zum Sammelbehälter (28) wird unterbrochen.

## Patentansprüche

1. Verfahren zur Herstellung wässriger Spülflüssigkeiten für das Spülen von Lackieranlagen für Wasserlacke durch Wiederaufbereitung der gebrauchten Spülflüssigkeiten und Überwachung des Recyclates auf das Vorhandensein von störenden Kontaminationen durch lackunverträgliche Begleitsubstanzen,
**dadurch gekennzeichnet, dass**
einer abgemessenen Probe der Recyclatflüssigkeit portionsweise VE-Wasser oder Wasser einer üblichen Trinkwasserqualität bis zu einem Gesamtwassergehalt der Probeflüssigkeit von 95 Gew.-% zugesetzt und die Probeflüssigkeit während des Wasserzusatzes auf das Auftreten einer Trübung geprüft werden, wobei das Auftreten einer Trübung der Probeflüssigkeit als Indikator für das Vorhandensein lackunverträglicher Kontamination steht.

2. Verfahren nach Anspruch 1
**dadurch gekennzeichnet, dass**
während des VE-Wasserzusatzes zur Probe eines bestimmten Recyclates eine einen Höchstwert erreichende auftretende Trübung visuell oder meßtechnisch ermittelt und der VE-Wasserzusatz bei der betrieblichen Recyclatprüfung auf diesen Höchstwert eingestellt werden.

3. Verfahren nach Anspruch 1 oder 2
**dadurch gekennzeichnet, dass**
die Menge des der Recyclatprobe zur Prüfung zugesetzten VE-Wassers, abhängig vom Ausgangswassergehalt der jeweiligen Recyclatprobe, jeweils so bemessen wird, dass einem Mengenanteil Recyclat bei einem Ausgangswassergehalt von ≥ 85 Gew.-% ein Mengenanteil VE-Wasser zur Prüfung zugesetzt, einem Mengenanteil Recyclat bei einem Ausgangswassergehalt von > 60 Gew.-% bis < 85 Gew.-% zwei Mengenanteile VE-Wasser zur Prüfung zugesetzt, einem Mengenanteil Recyclat bei einem Ausgangswassergehalt von > 40 Gew.-% bis ≤ 60 Gew.-% drei Mengenanteile VE-Wasser zur Prüfung zugesetzt, und einem Mengenanteil Recyclat bei einem Ausgangswassergehalt von ≤ 40 Gew.-% vier Mengenanteile VE-Wasser zur Prüfung zugesetzt werden, wobei die Gesamtmenge der jeweiligen Probesubstanz, bestehend aus Spülflüssigkeit und zugesetztem VE-Wasser, so eingestellt wird, dass sie dem Füllinhalt der Meßstrecke des Trübungsmeßgerätes entspricht.

4. Verfahren nach einem der Ansprüche 1 bis 3
**dadurch gekennzeichnet, dass**
die Beprobung und Untersuchung des erzeugten Recyclates während des Wiederaufbereitungsprozesses kontinuierlich und automatisch durchgeführt wird.

5. Verfahren nach einem der Ansprüche 1 bis 4
**dadurch gekennzeichnet, dass**
die während eines Wiederaufbereitungsprozesses anfallenden einzelnen Teilfraktionen des Recyclatesjeweils getrennt auf das Vorhandensein lackunverträglicher Kontaminationen geprüft werden.

6. Verfahren nach einem der Ansprüche 1 bis 5
**dadurch gekennzeichnet, dass**
das zur Wiederverwendung bestimmte Recyclat aus nach dem VE-Wasserzusatz keine Trübung zeigenden Teilfraktionen zusammengesetzt wird und die eine Trübung zeigenden Teilfraktionen nachbearbeitet oder ausgeleitet werden.

7. Verfahren nach einem der Ansprüche 1 bis 6
**dadurch gekennzeichnet, dass**
das jeweilige Meßsignal des Trübungssensors bei der meßtechnisch ermittelten Bestimmung der Trübung direkt auf die Prozeßsteuerung der Wiederaufbereitungsanlage einwirkt, um kontaminiertes Produkt auszuleiten und/oder die Einstellung der Prozeßparameter des Wiederaufbereitungsprozesses in einer gewünschten Weise zu ändern.

8. Verfahren nach einem der Ansprüche 1 bis 7
**dadurch gekennzeichnet, dass**
die Wiederaufbereitung der gebrauchten wässrigen Spülflüssigkeiten mittels kontinuierlicher Destillation mit fraktionierter Kondensation durchgeführt wird.

9. Verfahren nach Anspruch 8
**dadurch gekennzeichnet, dass**
die störende, lackunverträgliche Kontaminationen enthaltenden Destillatfraktionen durch eine oder mehrere Membrantrennanlagen gereinigt werden.

10. Verfahren nach einem der Ansprüche 1 bis 9
**dadurch gekennzeichnet, dass**
zur Prüfung auf lackunverträgliche Kontaminationen nach einer vorhergehenden Vergleichsprüfung mit VE-Wasser dem Recyclat Wasser einer üblichen Trinkwasserqualität zugesetzt wird.

11. Vorrichtung zur Herstellung wässriger Spülflüssigkeiten für das Spülen von Lackieranlagen für Wasserlacke durch Wiederaufbereitung der gebrauchten Spülflüssigkeiten zur Verhinderung der Einbringung störender lackunverträglicher Substanzen in das gewonnene Recyclat,
**dadurch gekennzeichnet, dass**
eine oder mehrere einstellbare Probenahmeeinrichtungen (F, G, H, E) für zu untersuchende, rückgewonnene Recyclate (B, C, D) sowie für VE-Wasser (A) so angeordnet sind, dass sie selbsttätig jeweils eine voreingestellte Menge eines Recyclates und anschließend jeweils eine voreingestellte Menge VE-Wasser in ein nachgeschaltetes Mischgefäß (I) fördern, dem in Reihe ein Trübungsmeßgerät (L) mit dem Sensor (M) und ein Auffangbehälter (O) für die gebrauchte Probeflüssigkeit nachgeschaltet sind, wobei jeweils zwischen Mischgefäß (I) und Trübungsmeßgerät (L) sowie zwischen Trübungsmeßgerät (L) und Auffangbehälter (O) Absperrventile (K, N) angeordnet sind, die den Durchlauf der Flüssigkeitsprobe durch die Meßanordnung steuern.

12. Vorrichtung nach Anspruch 11
**dadurch gekennzeichnet, dass**
die Fördermengen der Dosiereinrichtungen (F, G, H, E) in Abhängigkeit vom Wassergehalt des jeweils beprobten Recyclates (B, C, D) so eingestellt sind, dass einem Mengenanteil Recyclat bei einem Ausgangswassergehalt von ≥ 85 Gew.-% ein Mengenanteil VE-Wasser zur Prüfung, einem Mengenanteil Recyclat bei einem Ausgangswassergehalt von > 60 Gew.-% bis < 85 Gew.-% zwei Mengenanteile VE-Wasser zur Prüfung, einem Mengenanteil Recyclat bei einem Ausgangswassergehalt von > 40 Gew.-% bis ≤ 60 Gew.-% drei Mengenanteile VE-Wasser zur Prüfung und einem Mengenanteil Recyclat bei einem Ausgangswassergehalt von ≤ 40 Gew.-% vier Mengenanteile VE-Wasser zur Prüfung zudosierbar sind, so daß die Gesamtmenge der jeweiligen aus Recyclatprobe und zugesetztem VE-Wasser bestehenden Probesubstanz, dem Füllinhalt der Meßstrecke des Trübungsmeßgerätes (L) entspricht.

13. Vorrichtung nach einem der Ansprüche 11 oder 12
**dadurch gekennzeichnet, dass**
die Dosierpumpen für Spülflüssigkeit (F, G, H) und für VE-Wasser (E), die Ventile (K, N) sowie der Mischer (I) mit einer Programmsteuerung zur selbsttätigen und kontinuierlichen Untersuchung der Spülflüssigkeiten verbunden sind.

14. Vorrichtung nach einem der Ansprüche 11 bis 13
**dadurch gekennzeichnet, dass**
die Dosierpumpen für Recyclat (F, G, H) und für VE-Wasser (E) durch Ansaugleitungen direkt mit den Sammelbehältern für die einzelnen, im Wiederaufbereitungsprozeß entstehenden Recyclatfraktionen und dem Vorratsbehälter für VE-Wasser verbunden sind.

15. Vorrichtung nach einem der Ansprüche 11 bis 14
**dadurch gekennzeichnet, dass**
. die Dosierpumpen für Recyclat (F, G, H) durch Ansaugleitungen mit den Sammelbehältern für die einzelnen Destillatfraktionen einer Anlage zur kontinuierlichen Destillation mit fraktionierter Kondensation verbunden sind.

16. Vorrichtung nach einem der Ansprüche 11 bis 14
**dadurch gekennzeichnet, dass**
. die Dosierpumpen für Recyclat (F, G, H) durch Ansaugleitungen mit den Sammelbehältern für die einzelnen Destillatfraktionen und Filterfraktionen einer kontinuierlichen Destillationsanlage mit fraktionierter Kondensation und nachgeschalteter Membrantrennanlage verbunden sind.

## Claims

1. Process for producing aqueous rinsing liquids for rinsing painting lines for water-based paints by recycling the used rinsing liquids and monitoring the recycled liquid for the presence of disruptive contamination from paint-incompatible accompanying substances,
**characterised in that**
demineralised water or water of a conventional drinking water quality is added in portions to a measured sample of the recycled liquid to give a total water content in the sample liquid of 95 wt.% and during the addition of water the sample liquid is tested for the occurrence of turbidity, the occurrence of turbidity in the sample liquid being an indicator for the presence of paint-incompatible contamination.

2. Process according to claim 1,
**characterised in that**
during the addition of demineralised water to the sample of a particular recycled liquid an occurring turbidity reaching a maximum value is determined visually or by measurement and the addition of demineralised water during operational testing of the recycled liquid is adjusted to this maximum value.

3. Process according to claim 1 or 2,
**characterised in that**
the amount of demineralised water added to the recycled liquid sample for testing, depending on the original water content of the recycled liquid sample, is calculated such that one part of demineralised water is added for testing to one part of recycled liquid with an original water content of ≥ 85 wt.%, two parts of demineralised water are added for testing to one part of recycled liquid with an original water content of > 60 wt.% to < 85 wt.%, three parts of demineralised water are added for testing to one part of recycled liquid with an original water content of > 40 wt.% to ≤ 60 wt.%, and four parts of demineralised water are added for testing to one part of recycled liquid with an original water content of ≤ 40 wt.%, wherein the total amount of test substance, consisting of rinsing liquid and added demineralised water, is adjusted so that it corresponds to the capacity of the measuring section of the turbidimeter.

4. Process according to one of claims 1 to 3,
**characterised in that**
the sampling and analysis of the recycled liquid that is produced take place continuously and automatically during the recycling process.

5. Process according to one of claims 1 to 4,
**characterised in that**
the individual fractions of recycled liquid produced during a recycling process are tested separately for the presence of paint-incompatible contamination.

6. Process according to one of claims 1 to 5,
**characterised in that**
the recycled liquid intended for reuse is collected from fractions displaying no turbidity after the addition of demineralised water and fractions displaying turbidity are reprocessed or discharged.

7. Process according to one of claims 1 to 6,
**characterised in that**
where the turbidity is determined by measurement, the measuring signal from the turbidity sensor acts directly on the process controller of the recycling plant to discharge contaminated product and/or to change the setting of the process parameters for the recycling process in a desired manner.

8. Process according to one of claims 1 to 7,
**characterised in that**
recycling of the used aqueous rinsing liquids is performed by means of continuous distillation with fractional condensation.

9. Process according to claim 8,
**characterised in that**
the distillate fractions containing the disruptive, paint-incompatible contamination are purified using one or more membrane separating plants.

10. Process according to one of claims 1 to 9,
**characterised in that**
water of a conventional drinking water quality is added to the recycled liquid after a previous comparative test with demineralised water to test for paint-incompatible contamination.

11. Device for producing aqueous rinsing liquids for rinsing paint lines for water-based paints by recycling the used rinsing liquids to prevent the introduction of disruptive paint-incompatible substances into the recycled liquid obtained,
**characterised in that**
one or more adjustable sampling devices (F, G, H, E) for recovered recycled liquids to be analysed (B, C, D) and for demineralised water (A) are arranged in such a way that they automatically convey a predefined amount of a recycled liquid followed by a predefined amount of demineralised water to a downstream mixing vessel (I), to which a turbidimeter (L) with sensor (M) and a receiving container (O) for the used sample liquid are connected in series, there being shut-off valves (K, N) positioned between the mixing vessel (I) and the turbidimeter (L) and between the turbidimeter (L) and the receiving container (O) which control the flow of the liquid sample through the measurement setup.

12. Device according to claim 11,
**characterised in that**
the delivery amounts from the metering devices (F, G, H, E) are adjusted according to the water content of the individual sampled recycled liquid (B, C, D) such that one part of demineralised water can be added for testing to one part of recycled liquid with an original water content of ≥ 85 wt.%, two parts of demineralised water can be added for testing to one part of recycled liquid with an original water content of > 60 wt.% to < 85 wt.%, three parts of demineralised water can be added for testing to one part of recycled liquid with an original water content of > 40 wt.% to ≤ 60 wt.%, and four parts of demineralised water can be added for testing to one part of recycled liquid with an original water content of ≤ 40 wt.%, such that the total amount of test substance, consisting of recycled liquid sample and added demineralised water, corresponds to the capacity of the measuring section of the turbidimeter (L).

13. Device according to one of claims 11 or 12,
**characterised in that**
the metering pumps for rinsing liquid (F, G, H) and for demineralised water (E), the valves (K, N) and the mixer (I) are connected to a program controller for the automatic and continuous analysis of the rinsing liquids.

14. Device according to one of claims 11 to 13,
**characterised in that**
the metering pumps for recycled liquid (F, G, H) and for demineralised water (E) are connected by suction lines directly to the collection containers for the individual recycled liquid fractions produced in the recycling process and to the storage container for demineralised water.

15. Device according to one of claims 11 to 14,
**characterised in that**
the metering pumps for recycled liquid (F, G, H) are connected by suction lines to the collection containers for the individual distillate fractions of a plant for continuous distillation with fractional condensation.

16. Device according to one of claims 11 to 14,
**characterised in that**
the metering pumps for recycled liquid (F, G, H) are connected by suction lines to the collection containers for the individual distillate fractions and filter fractions of a continuous distillation plant with fractional condensation and a downstream membrane separating plant.

## Revendications

1. Procédé de préparation de liquides de rinçage à base d'eau pour le rinçage d'installations de peinture pour peintures hydrodiluables grâce au traitement des liquides de rinçage et au contrôle dans les substances recyclées de présence de contaminations gênantes par des substances associées incompatibles avec la peinture,
**caractérisé en ce**
**qu'**à un échantillon dosé de liquide recyclé est ajoutée par parties de l'eau déminéralisée ou de l'eau de la qualité d'une eau potable habituelle jusqu'à une teneur en eau totale de l'échantillon de liquide de 95 % en poids et en ce qu'il est vérifié durant l'adjonction d'eau l'apparition d'un trouble dans l'échantillon de liquide, l'apparition d'un trouble dans l'échantillon de liquide faisant office d'indicateur de présence d'une contamination incompatible avec la peinture.

2. Procédé selon la revendication 1
**caractérisé en ce que**,
lors de l'adjonction d'eau déminéralisée dans l'échantillon d'une substance recyclée définie, on détecte, visuellement ou par une méthode de mesure, l'apparition d'un trouble correspondant à une valeur maximale atteinte et **en ce que** l'adjonction d'eau déminéralisée est réglée sur cette valeur maximale lors du contrôle d'exploitation des substances recyclées.

3. Procédé selon la revendication 1 ou 2
**caractérisé en ce que**
la quantité d'eau déminéralisée, qui dépend de la teneur en eau initiale de l'échantillon de substance recyclée concerné, ajoutée à l'échantillon de substance recyclée à fins de contrôle est mesurée de telle manière qu'on ajoute à un volume de substance recyclée pour une teneur en eau initiale ≥ 85 % en poids un volume d'eau déminéralisée à fins de contrôle, qu'on ajoute à un volume de substance recyclée pour une teneur en eau initiale > 60 % en poids et < 85 % en poids deux volumes d'eau déminéralisée à fins de contrôle, qu'on ajoute à un volume de substance recyclée pour une teneur en eau initiale > 40 % en poids et < 60 % en poids trois volumes d'eau déminéralisée à fins de contrôle et qu'on ajoute à un volume de substance recyclée pour une teneur en eau initiale ≤ 40 % en poids quatre volumes d'eau déminéralisée à fins de contrôle, la quantité totale de substance de l'échantillon concerné, composée de liquide de rinçage et d'eau déminéralisée ajoutée, étant calculée de manière à correspondre au contenu de la distance mesurée du turbidimètre.

4. Procédé selon l'une des revendications 1 à 3
**caractérisé en ce que**
l'échantillonnage et l'examen de la substance recyclée produite s'effectuent en continu et de manière automatique durant le processus de traitement.

5. Procédé selon l'une des revendications 1 à 4
**caractérisé en ce que**
les différentes fractions produites durant un processus de traitement font l'objet de contrôles séparés concernant la présence de contaminations incompatibles avec la peinture.

6. Procédé selon l'une des revendications 1 à 5
**caractérisé en ce que**
la substance recyclée destinée au traitement se compose de fractions ne présentant plus de trouble après adjonction d'eau déminéralisée et **en ce que** les fractions présentant un trouble sont traitées ou évacuées.

7. Procédé selon l'une des revendications 1 à 6
**caractérisé en ce que**
le signal de mesure concerné du capteur de turbidité agit directement sur la commande du process de l'installation de traitement lors de la détermination du trouble définie par une méthode de mesure afin d'évacuer le produit contaminé et/ou de modifier d'une manière souhaitable le réglage des paramètres du process de traitement.

8. Procédé selon l'une des revendications 1 à 7
**caractérisé en ce que**
le traitement des liquides de rinçage usés à base d'eau s'effectue par distillation continue avec condensation fractionnée.

9. Procédé selon la revendication 8
**caractérisé en ce que**
les fractions de distillat présentant des contaminations gênantes incompatibles avec la peinture sont purifiées par une ou plusieurs installations de séparation à membrane.

10. Procédé selon l'une des revendications 1 à 9
**caractérisé en ce que**
de l'eau de la qualité d'une eau potable habituelle est ajoutée à la substance recyclée à fins de contrôle de contaminations incompatibles avec la peinture selon un examen comparatif préalable avec de l'eau déminéralisée.

11. Dispositif de préparation de liquides de rinçage à base d'eau pour le rinçage d'installations de peinture pour peintures hydrodiluables par traitement des liquides de rinçage usés afin d'éviter l'apport de substances gênantes incompatibles avec la peinture dans le produit de recyclage obtenu
**caractérisé en ce**
**qu'**un ou plusieurs dispositifs réglables de prélèvements d'échantillons (F, G, H, E) pour les substances recyclées obtenues (B, C, D) à tester et pour eau déminéralisée (A) sont placés de manière à pouvoir envoyer automatiquement une quantité prédéterminée de substance recyclée, puis une quantité prédéterminée d'eau déminéralisée dans un mélangeur (I) situé en aval à la suite duquel sont installés en série un turbidimètre (L) avec capteur (M) et un collecteur (O) pour l'échantillon de liquide usé, des soupapes d'arrêt (K, N) étant placées entre le mélangeur (I) et le turbidimètre (L) ainsi qu'entre le turbidimètre (L) et le collecteur (O) afin de réguler l'écoulement de l'échantillon de liquide dans le dispositif de mesure.

12. Dispositif selon la revendication 11
**caractérisé en ce que**
les débits des dispositifs de dosage (F, G, H, E) sont réglés en fonction de la teneur en eau de la substance recyclée testée (B, C, D) de manière à ce qu'il soit possible de doser un volume d'eau déminéralisée pour une teneur en eau initiale ≥ 85 % en poids pour un volume de substance recyclée à fins de contrôle, de doser deux volumes d'eau déminéralisée pour une teneur en eau initiale > 60 % en poids et < 85 % en poids pour un volume de substance recyclée à fins de contrôle, de doser trois volumes d'eau déminéralisée pour une teneur en eau initiale > 40 % en poids et < 60 % en poids pour un volume de substance recyclée à fins de contrôle et de doser quatre volumes d'eau déminéralisée pour une teneur en eau initiale ≤ 40 % en poids pour un volume de substance recyclée à fins de contrôle, si bien que la quantité totale de substance de l'échantillon de substance recyclée concerné composé de l'échantillon de substance recyclée et de l'eau déminéralisée ajoutée, corresponde au contenu de la distance mesurée du turbidimètre (L).

13. Dispositif selon l'une des revendications 11 ou 12
**caractérisé en ce que**
les pompes de dosage du liquide de rinçage (F, G, H) et de l'eau déminéralisée (E), les soupapes (K, N) ainsi que le mélangeur (I) sont liés à une commande programmable afin de contrôler en continu de manière automatique les liquides de rinçage.

14. Dispositif selon l'une des revendications 11 à 13
**caractérisé en ce que**
les pompes de dosage de la substance recyclée (F, G, H) et de l'eau déminéralisée (E) sont reliées directement par des tuyaux d'admission aux récipients collecteurs des différentes fractions de substance recyclée obtenues par traitement et au réservoir d'eau déminéralisée.

15. Dispositif selon l'une des revendications 11 à 14
**caractérisé en ce que**
les pompes de dosage de la substance recyclée (F, G, H) sont reliées par des tuyaux d'admission aux récipients collecteurs des différentes fractions de distillat d'une installation de distillation en continu à condensation fractionnée.

16. Dispositif selon l'une des revendications 11 à 14
**caractérisé en ce que**
les pompes de dosage de la substance recyclée (F, G, H) sont reliées par des tuyaux d'admission aux récipients collecteurs des différentes fractions de distillat et fractions de filtrage d'une installation de distillation en continu à condensation fractionnée et avec une installation de séparation à membrane en aval.
